(19) Europäisches Patentamt
European Patent Office
Office européen des brevets

(11) Veröffentlichungsnummer: **0 545 972 B1**

(12) **EUROPÄISCHE PATENTSCHRIFT**

(45) Veröffentlichungstag der Patentschrift: **02.11.94**

(51) Int. Cl.5: **A61K 37/02**, A61K 31/35

(21) Anmeldenummer: **91914735.5**

(22) Anmeldetag: **20.08.91**

(86) Internationale Anmeldenummer:
**PCT/EP91/01580**

(87) Internationale Veröffentlichungsnummer:
**WO 92/03146 (05.03.92 92/06)**

# (54) THERAPEUTISCH WIRKSAMES STOFFGEMISCH AUS GLUTATHION- UND ANTHOCYAN-VERBINDUNGEN.

(30) Priorität: **20.08.90 DE 4026263**

(43) Veröffentlichungstag der Anmeldung:
**16.06.93 Patentblatt 93/24**

(45) Bekanntmachung des Hinweises auf die Patenterteilung:
**02.11.94 Patentblatt 94/44**

(84) Benannte Vertragsstaaten:
**AT BE CH DE DK ES FR GB GR IT LI LU NL SE**

(56) Entgegenhaltungen:
**WO-A-89/00427**
**DE-A- 2 740 346**
**DE-A- 3 615 313**

(73) Patentinhaber: **OHLENSCHLÄGER, Gerhard, Dr. med.**
**Hauptstrasse 22**
**D-61462 Königstein (DE)**

Patentinhaber: **Treusch, Gernot**
**Offenbacher Landstrasse 416/418**
**D-60599 Frankfurt (DE)**

(72) Erfinder: **OHLENSCHLÄGER, Gerhard, Dr. med.**
**Hauptstrasse 22**
**D-61462 Königstein (DE)**
Erfinder: **Treusch, Gernot**
**Offenbacher Landstrasse 416/418**
**D-60599 Frankfurt (DE)**

(74) Vertreter: **Lieck, Hans-Peter, Dipl.-Ing.**
**Lieck, Endlich & Partner**
**Widenmayerstrasse 36**
**D-80538 München (DE)**

EP 0 545 972 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die wissenschaftlichen Erkenntnisse der letzten Jahre zeigen, daß zerstörerische physikalisch-chemische Prozesse durch "freie" Radikale, Radikal-Kettenreaktionen und/oder durch aktivierte Sauerstoff-Stufen einen immer wichtiger werdenden Stellenwert in der Pathogenese vieler akuter und vor allem chronischer Erkrankungen einnehmen, wobei u.a. arterielle und venöse Angiopathien, Allergien, Autoaggressionskrankheiten und Tumorerkrankungen zu nennen sind. Reaktionen von "freien" Radikalen und aktivierten Sauerstoff-Stufen (ASS), aber auch von Radikalen, die durch ionisierende Strahlung bei der Wasser-Radiolyse entstehen, führen ständig zu Veränderungen und Zerstörungen an den Biomolekülen (DNA, RNA, Enzym- und Strukturproteinen, ungesättigten Fettsäuren u.a.), sowie an allen Zellen und Zellorganellen über radikalische Reaktionsphänomene der Lipidperoxidation auch zu Membranschäden und Membranzerstörungen. Radikalische Prozesse sind in die Ätiologie aller Erkrankungen eingeschaltet; oft sind sie sogar die Ursache dieser Krankheiten oder unterhalten diese durch radikalische Kettenreaktionen.

Mensch und Tiere werden aus diesem Grunde durch bestimmte antioxidativ wirkende Enzyme, wie Superoxid-Dismutasen, Catalasen und Peroxidasen geschützt, welche die aktivierten Sauerstoff-Stufen "entschärfen".

Es ist bekannt (WO 89/00427), daß zur Erhaltung der Funktionalität vieler, vielleicht aller Enzyme des Zellstoffwechsels, zur Verhinderung oxidativer Veränderungen ihrer katalytischen und allosterischen Zentren und zur Erhaltung einer optimalen Konformation das reduktive Potential des reduzierten Glutathions, d.h. seine optimale, hohe intrazelluläre Konzentration von äußerster Wichtigkeit ist und durch Gaben von reduziertem Glutathion erhöht oder - im Falle eines gestörten Zellstoffwechsels - jedenfalls wieder hergestellt werden kann.

Es ist ebenfalls bekannt (WO 89/00427), daß es günstiger sein kann, statt des reduzierten Glutathions selber ein Thiol-Derivat des Glutathions dem Körper von außen zuzuführen. Die Thiol-Derivate zeichnen sich durch eine besonders gute Bioverfügbarkeit aus. Ihre Permeationsfähigkeit durch biologische Membranen ist hoch. Die für den therapeutischen Effekt wichtige SH-Gruppe des Glutathions ist auf dem Weg durch biologische Kompartimente bis hin zum gewünschten Wirkort geschützt und sie verursachen keine Hemmung der in die endogene Glutathion-Biosynthese eingeschalteten Enzyme.

Es sind auch bereits Arzneimittel bekannt (DE-OS 27 40 346), die als aktiven Bestandteil ein Anthocyanidin, z.B. Cyanidin, Peonidin, Delphinidin, Petunidin, Pelargonidin und/oder Malvidin, enthalten. Diese Arzneimittel sollen zur Behandlung von Wunden, Geschwuren, Entzündungszuständen und pathogenen Zuständen des Gefäßsystems oder Störungen verabreicht werden, die durch Verschlechterung des Lipoid- oder Glycidstoffwechsels verursacht werden.

Obwohl reduziertes Glutathion als Coenzym (Selen-abhängige Glutathion-Peroxidasen) und als Cofaktor (Glutathion-S-Transferasen) sowie auch als nicht-enzymatischer Scavenger und nukleophile Substanz in der Lage ist, direkt elektrophile Xenobiotika als primäre oder sekundäre Radikale sowie solche Radikale zu detoxifizieren, die im Zellstoffwechsel durch Exposition gegenüber energiereichen Strahlen entstanden sind, kann es bei alleiniger therapeutischer Anwendung von reduziertem Glutathion - auch bei ausreichender Applikation desselben - zu Mangelzuständen kommen. Diese erklären sich durch:

- Schlechte genetische Ausstattung an antioxidativen, d.h. Scavenger-Enzymen (Enzymopathien);
- mangelhafte Biosynthese antioxidativer Enzyme in verschiedenen Kompartimenten und in Abhängigkeit von bestimmten, ungünstigen Lebensphasen (Enzymopenien);
- "oxidativer Verschleiß" von reduziertem Glutathion unter besonderen Umständen, wie Intoxikationen, Entzündungen, Infekten, Mängeln an nicht-enzymatischen oder enzymatischen Scavengern, mit der extrem ungünstigen Möglichkeit der Entstehung von Thyil-Radikalen, Glutathion-Disulfid-Anion-Radikalen oder auch von Glutathion-Peroxysulphenyl-Radikalen;
- de-novo-Biosynthesestörungen von endogenem reduzierten Glutathion;
- Hochleistungssport, Kachexien, aufzehrende Erkrankungen, Alter.

Alle diese unterschiedlich zustande kommenden Mangelzustände führen zu Veränderungen des in allen biologischen Räumen lebender Systeme vorliegenden negativen Redoxpotentials und über Entgleisungen des "Redoxverschiebe-Systems"

Reduziertes Glutathion $\rightleftharpoons$ gemischte Disulfide $\rightleftharpoons$
oxidiertes Glutathion

zu Enzymstörungen, vor allem in antioxidativen Enzymen und Reparaturenzymen, zur Zellstoffwechsel-Entgleisung, zu Mutationen, zur malignen Transformation oder auch zur Zellnekrose.

Es wurde nun erkannt, daß sich die therapeutischen Mißerfolge, die sich bei alleinigem Einsatz von reduziertem Glutathion aus den genannten pathobiochemischen Störungen ergeben, durch eine Kombination von reduziertem Glutathion mit Anthozyanen verhindert werden können, wobei anstelle des reduzierten Glutathions oder zusätzlich zu diesem auch die Thiol-Derivate des Glutathions mit Vorteil einsetzbar sind.

Somit stellt die Erfindung ein Stoffgemisch zur therapeutischen Behandlung des menschlichen oder tierischen Körpers zur Verfügung, das reduziertes Glutathion und mindestens eine Anthocyan-Verbindung der Gruppe Pelargonidin, Päonidin, Cyanidin, Melvidin, Petunidin, Delphinidin enthält, wobei das reduzierte Glutathion ganz oder teilweise durch mindestens ein Thiol-Derivat des Glutathions der Gruppe Methyl-Glutathionyl-(Thio)-Äther, Äthyl-Glutathionyl-(Thio)-Äther, Mono-Acetyl-Glutathionyl-(Thio)-Ester, Mono-Phosphorsäure-Glutathionyl-(Thio)-Ester ersetzt sein kann.

Reduziertes Glutathion ist ein in seiner reduzierten Form (G-SH) in den meisten Zellen von Mensch und Säugetieren vorkommendes Tripeptid, das aus den drei Aminosäuren Glutaminsäure, Cystein und Glycin besteht und folgende Strukturformel hat:

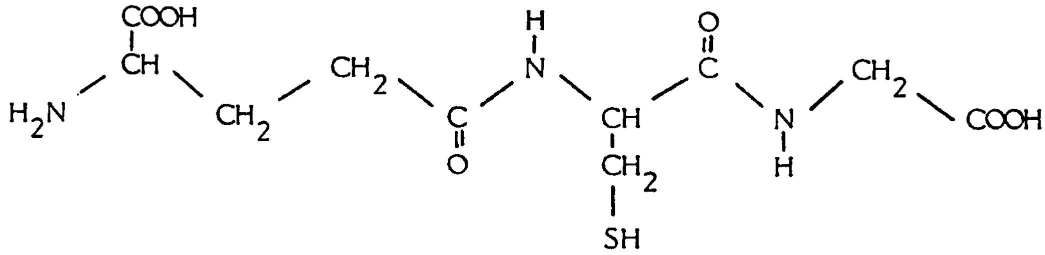

reduziertes Glutathion (gamma-Glutamyl-cysteinyl-glycin).

Die erfindungsgemäß einsetzbaren Thiol-Derivate des Glutathions haben die Strukturformel

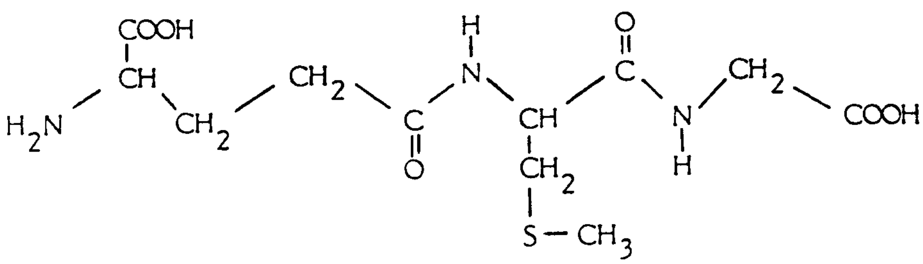

Methyl-glutathionyl-(thio)-äther bzw. Monomethyl-thioester

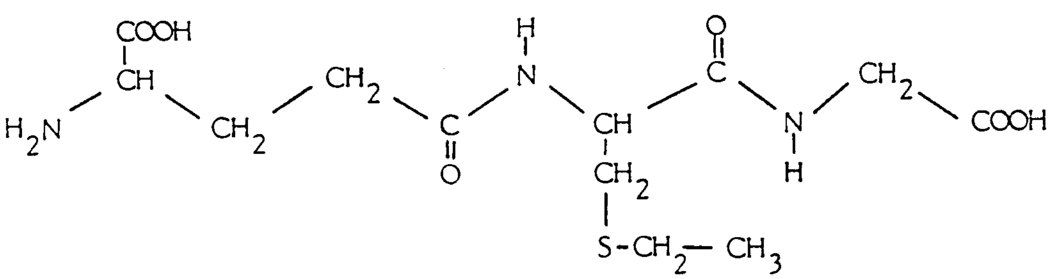

Äthyl-glutathionyl-(thio)-äther bzw. Monoäthyl-thioester

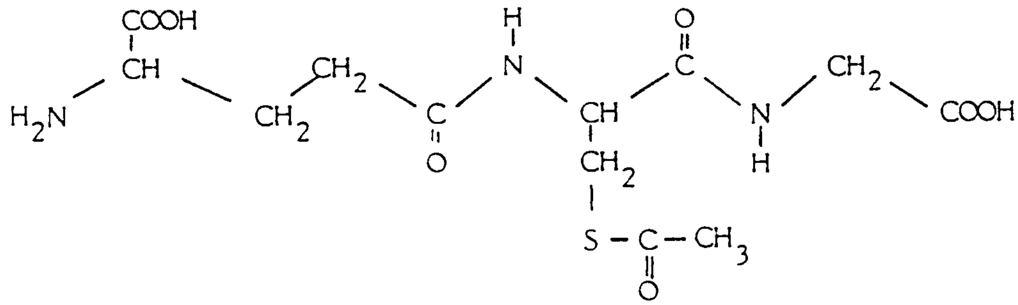

Mono-acetyl-glutathionyl-(thio)-ester bzw. Monoacetyl-thio-ester

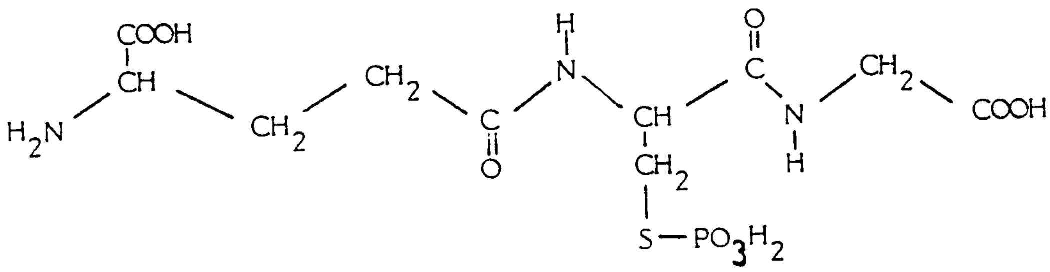

Mono-phosphorsäure-glutathionyl-(thio)-ester bzw. Monophosphorsäure-thioester

Anthocyane kommen in vielen höheren Pflanzen vor und sind dort für die rote, violette, blaue oder blauschwarze Farbe von Blüten und Früchten verantwortlich. Es handelt sich um heterocyclische 2-Phenylchromenol-Mehrringsysteme mit unterschiedlichen Hydroxylierungsmustern und unterschiedlichen Absorptionsspektren im sichtbaren Licht. Die zuckerfreien Aglykonkomponenten der Anthocyane bezeichnet man als Anthocyanidine. Sie können leicht durch Hydrolyse von den in üblichen Früchten enthaltenen Glykosiden erhalten werden (vgl. DE-OS 27 40 346) und haben folgende Strukturformel:

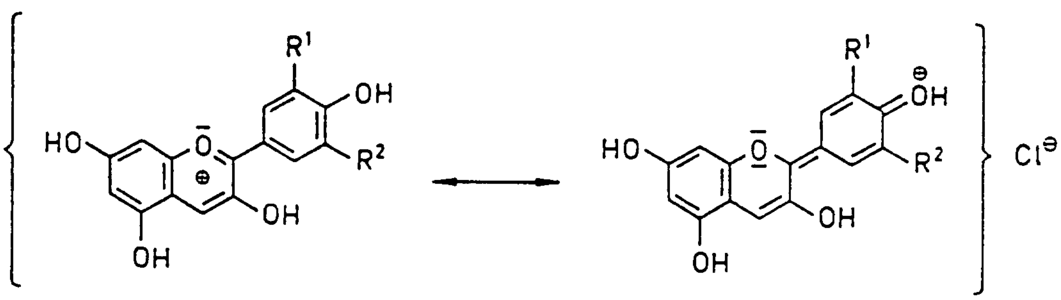

| $R^1$ | $R^2$ | Trivialname | $\lambda_{max}$ (nm) (Farbe) |
|---|---|---|---|
| H | H | Pelargonidin | 520 (rotorange) |
| H | $OCH_3$ | Päonidin | 532 (rotviolett) |
| H | OH | Cyanidin | 535 (rotviolett) |
| $OCH_3$ | $OCH_3$ | Malvidin | 542 (violettrot) |
| OH | $OCH_3$ | Petunidin | 543 (violettrot) |
| OH | OH | Delphinidin | 544 (blauviolett) |

Anthocyane können als gute Scavenger für das Superoxid-Anion-Radikal ($O_2^{\cdot}$ ), für Wasserstoffperoxid ($H_2O_2$), für das Hydroxyl-Radikal (OH•), für Alkoxyl-Radikale (LO•), Peroxyl-Radikale (L O O•), für

Singulettsauerstoff ($O_2(^1\Delta_g)$ und viele andere Radikale fungieren. Anthocyane können aber auch als photobiologische Inhibitoren regelnd und detoxifizierend in über Sauerstoff ablaufende sensibilisierte Photoreaktionen eingreifen und so zell- und molekül-schädigende Radikal- und Radikalkettenreaktionen verhindern, unabhängig davon, auf welche Weise diese entstanden sind.

Anthocyane schützen vor zelltoxischen und kanzerogenen Aldehyden (4-Hydroxy-hexenal, 4-Hydroxy-octenal, 4-Hydroxy-nonenal, Propanal, Butanal, Pentanal, Hexanal, 2,4-Heptadienal, Malondialdehyd, u.a.). Sie verhindern sogar ihre Bildung im Rahmen lipidperoxidativer Kettenreaktionen. Auch detoxifizieren sie den beim Äthanolabbau entstehenden Acetaldehyd und den beim Methanolabbau entstehenden oder auf anderen Wegen inkorporierten Formaldehyd.

Die Anthocyan-Verbindungen und reduziertes Glutathion und/oder seine Thiol-Derivate ergänzen sich dosisabhängig im therapeutischen Einsatz optimal bei vielen Zellstörungen und vielen Zell- und Enzym-Regulationsstörungen. Neben dem qualitativen therapeutischen Aspekt ist bei dem kombinierten Einsatz von reduziertem Glutathion und/oder seinen Thiol-Derivaten zusammen mit Anthocyan-Verbindungen vor allem die Quantenausbeute hinsichtlich der Scavengerfunktion wesentlich effektiver. Thyil-Radikale (GS•), Glutathion-Disulfid-Anion-Radikale (G-S-S-G•) und auch Glutathion-Peroxysulphenyl-Radikale (G-S 0 0•) werden entweder in ihrer Entstehung verhindert oder detoxifiziert.

Außerdem kommt es nicht nur zu einer Reduzierung von oxidiertem Glutathion (G-S-S-G) und zu einer Verhinderung und/oder zu einer Detoxifikation von Glutathion-Radikalen, sondern sogar in einem oszillierenden Reaktionszyklus zwischen beiden Stoffen bzw. Stoffgruppen (Glutathion und Anthocyane) zu einer anhaltenden Regeneration der radikal-detoxifizierenden Funktionen.

Die gegenseitige Ergänzung der beiden Stoffgruppen in dem erfindungsgemäßen Stoffgemisch ist so optimal, daß das reduzierte Glutathion seinen lebenswichtigen Regelfunktionen auf genetischer und auf Enzym-Ebene und schließlich auf allen Ebenen wieder voll nachkommen kann.

Reduziertes Glutathion (G-SH) reagiert u.a. mit Chinonen unter Bildung von Glutathionyl-Hydrochinon-Konjugaten, die zu den korrespondierenden Hydrochinonen autoxidieren können. Die G-SH-Konjugation vermindert den radikalischen, elektrophilen Charakter von Chinonen bei Verbesserung ihrer Hydrophilie. Solche Konjugatbildungen, die von großem toxikologischem Interesse sind, werden aber sowohl von der Chinon-, als auch von der G-SH-Konzentration, intrazellulär limitiert. Das ist ein weiterer therapeutischer Ansatzpunkt für viele phänomenologisch so unterschiedlich erscheinende Krankheiten. Vor allem zeigt die Kombination von reduziertem Glutathion oder seinen Thiol-Derivaten mit Anthocyan-Verbindungen neben der Neueinstellung eines physiologischen Regelkreisverhaltens auf allen biologischen Ebenen und in allen Kompartimenten lebender Systeme auch therapeutische Wirkungen bei auf unterschiedlichen Wegen initiierten Radikal- und Radikalkettenreaktionen (thermisch, chemisch, mechanisch, infektiös-toxisch, strahlenbedingt u.a.). und bei dem pathobiochemisch so wichtigen Phänomen der Lipidperoxidation.

Das erfindungsgemäße Stoffgemisch verhindert weitgehend Vernetzungen (cross-links) von Biomolekülen (Bindegewebe, Proteine, DNA u.a.) beim Diabetes mellitus und besonders auch beim Diabetischen Spätsyndrom (Verhinderung von Amadorikörpern). Sie verhindert weiterhin lipidperoxidativ entstandene und pathogenetisch verursachte polyneuropathische Degenerationen am peripheren und zentralen Nervensystem im Sinne der Verhinderung von Lipofuscinbildung oder Lipofuscinherden. Außerdemn kommt es gerade durch die Kombination von Glutathion- und Anthocyan-Verbindungen zur Verhinderung von Proteindenaturierungen an bradytrophen Geweben, wie z.B. an Cornea, Augenlinse und Glaskörper des Auges.

Eine weitere Steigerung der Wirksamkeit ergibt sich durch Hinzunahme von Vitamin E (Alpha-Tokopherolazetat) und/oder Vitamin A und/oder $\beta$-Carotin und/oder Selen und/oder L-Cystein in das erfindungsgemäße Stoffgemisch.

Besonders geeignet ist eine orale Verabreichung des erfindungsgemäßen Stoffgemisches, wobei die therapeutische Dosierung im Bereich von 50 mg bis 2400 mg pro Tag liegt.

Das erfindungsgemäße Stoffgemisch hat dementsprechend ein breites pharmakologisches und therapeutisches Anwendungsgebiet. Sein Einsatz kommt u.a. in Frage

- zur Behandlung von Krebserkrankungen jeder Genese, auch für maligne Erkrankungen der Blutzellen und ihrer Vorstufen,
- zur Substitution und Stoffwechselregulation bei Durchführung anderer Krebstherapien, Chemotherapien, Strahlentherapien und/oder naturheilkundlicher Therapien,
- zur Prophylaxe und Behandlung von Metastasen im Rahmen maligner Tumorerkrankungen,
- zur Behandlung von Hepatopathien, insbesondere akute und chronische Erkrankungen an Hepatitis wie chemisch-toxische und infektiös-toxische Hepatitis, Virus-, Rickettsien-, Bakterien- oder Protozoen-bedingte Hepatitiden sowie chronisch-aggressive Hepatitis, Fettleber, Fettzirrhose und Leberzirrhosen jeder Genese,

- zur Behandlung von Störungen der immunologischen Abwehrfunktion im Bereich der Natural-Killer-Zellen, Monozyten, Makrophagen, Granulozyten, T- und B-Lymphozyten, Plasmazellen, sowie Störungen der Komplementfaktoren und Antikörpersynthese,
- zur Behandlung komplexer Störungen der Lymphokon-Biosynthese in T-Helferzellen, Makrophagen und anderen Zellen,
- zur Behandlung von Cardiomyopathien jeder Genese, auch in Verbindung mit anderen Therapien, alle Formen von Koronarleiden, Angina pectoris, Myokardinfarktprophylaxe sowie Notfalltherapie des Herzinfarktes in Verbindung mit anderen Notfallmedikamenten,
- zur Behandlung erworbener und auch angeborener Formen von Skelettmuskelstörungen,
- zur Behandlung neurologischer Erkrankungen entzündlicher, allergischer oder degenerativer Genese,
- zur Behandlung aller Formen von Blutzellerkrankungen, Anämien, Leukopenien, Lymphopenien und Thrombozytophenien,
- zur Prophylaxe von Augenlinsenschäden, toxischen Störungen der Netzhaut und des Glaskörpers, sowie zur Kataraktprophylaxe,
- zur Behandlung aller Formen der Überoxidation bzw. des oxidativen Stresses, beispielsweise im Rahmen der Anwendung von Sauerstoff-Therapien oder Therapien mit aktivierten Sauerstoff-Stufen (Sauerstoff-Radikale) sowie zum Schutz bei der Anwendung von hyperbarer Sauerstofftherapie, Sauerstoff-Mehrschritt-Therapie, bei Ozontherapien und HOT-Therapien,
- zu Intoxikationen, die im menschlichen Organismus über radikalische Kettenreaktionen zu Biomolekül- und Gewebeschäden führen,
- zur begleitenden Therapie bei Bestrahlungsbehandlungen, Cytostatikabehandlungen sowie zur Abschwächung oder Verhinderung des Übelseins, der Nausea u.a.,
- nach Narkosen, insbesondere nach Vollnarkosen bei herz- und lebergeschädigten Patienten,
- zur Intoxikationen mit Xenobiotica, insbesondere mit toxischen Spurenelementen und mit Schwermetallen,
- zur Behandlung von Proliferationsstörungen und Differenzierungsstörungen von Epithel-, Endothel- und Schleimhautgeweben,
- zur Behandlung der pathophysiologischen und unterschiedlich entstehenden Arteriosklerose,
- zur Basisbehandlung und adjuvanten Behandldung von Allergien,
- zur Behandlung der Impotentia coeundie und Impotentia generandi, sowie Fertilitätsstörungen und Potenzstörungen jeder Genese,
- bei vorzeitigem Altern, beim Altersverschleiß aller Gewebe, auch des Haut- und Hautbindegewebes und zur Prophylaxe bei Betätigungen und Lebensgewohnheiten, die zu vorzeitigem Altern oder zu vorzeitigem Organ- und Gewebeverschleiß führen.

## Patentansprüche

**1.** Stoffgemisch, enthaltend
reduziertes Glutathion
und mindestens eine Anthocyan-Verbindung aus der Gruppe
Pelargonidin
Päonidin
Cyanidin
Malvidin
Petunidin
Delphinidin
zur therapeutischen Behandlung des menschlichen oder tierischen Körpers.

**2.** Stoffgemisch, enthaltend
mindestens ein Thiol-Derivat des Glutathions aus der Gruppe
Methyl-Glutathionyl-(Thio)-Äther
Äthyl-Glutathionyl-(Thio)-Äther
Mono-Acetyl-Glutathionyl-(Thio)-Ester
Mono-Phosphorsäure-Glutathionyl-(Thio)-Ester
und mindestens eine Anthocyan-Verbindung aus der Gruppe
Pelargonidin
Päonidin
Cyanidin

Malvidin
Petunidin
Delphinidin
zur therapeutischen Behandlung des menschlichen oder tierischen Körpers.

**3.** Stoffgemisch, enthaltend
reduziertes Glutathion und mindestens ein Thiol-Derivat des Glutathions aus der Gruppe
Methyl-Glutathionyl-(Thio)-Äther
Äthyl -Glutathionyl-(Thio)-Äther
Mono-Acetyl-Glutathionyl-(Thio)-Ester
Mono-Phosphorsäure-Glutathionyl-(Thio)-Ester
und mindestens eine Anthocyan-Verbindung aus der Gruppe
Pelargonidin
Päonidin
Cyanidin
Malvidin
Petunidin
Delphinidin
zur therapeutischen Behandlung des menschlichen oder tierischen Körpers.

**4.** Stoffgemisch nach Anspruch 1, 2 oder 3, das zusätzlich Vitamin E und/oder Vitamin A enthält.

**5.** Stoffgemisch nach Anspruch 1, 2, 3 oder 4, das zusätzlich $\beta$ -Carotin enthält.

**6.** Stoffgemisch nach Anspruch 1, 2, 3, 4 oder 5, das zusätzlich Selen enthält.

**7.** Stoffgemisch nach Anspruch 1, 2, 3, 4, 5 oder 6, das zusätzlich L-Cystein enthält.

**8.** Stoffgemisch nach einem der Ansprüche 1 bis 7 als Radikalfänger (nicht-enzymatischer Scavenger) und Radikalketten-Terminator zur Behandlung von Erkrankungen, die mit einer Hyperradikalie, mit dem Phänomen des "oxidativen Stresses" (Überoxidation) und/oder mit Enzymopenien und Enzympathien anti-oxidativ wirkender Enzyme einhergehen.

## Claims

**1.** A mixture containing
reduced glutathion
and at least one anthocyan compound from the group
pelargonidine
peonidine
cyanidine
malvidine
petunidine
delphinidine
for the therapeutic treatment of the human or animal body.

**2.** A mixture containing
at least one thiol-derivative of glutathion from the group
methyl-glutathionyl-(thio)-ether
ethyl-glutathionyl-(thio)-ether
mono-acetyl-glutathionyl-(thio)-ester
mono-phosphoric acid-glutathionyl-(thio)-ester
and at least one anthocyan compound from the group
pelargonidine
peonidine
cyanidine
malvidine
petunidine

delphinidine
for the therapeutic treatment of the human or animal body.

3. A mixture containing
reduced glutathion
and at least one thiol-derivative of glutathion from the group
methyl-glutathionyl-(thio)-ether
ethyl-glutathionyl-(thio)-ether
mono-acetyl-glutathionyl-(thio)-ester
mono-phosphoric acid-glutathionyl-(thio)-ester and at least one anthocyan compound from the group
pelargonidine
peonidine
cyanidine
malvidine
petunidine
delphinidine
for the therapeutic treatment of the human or animal body.

4. A mixture according to claim 1, 2 or 3, which additionally contains vitamin E and/or vitamin A.

5. A mixture according to claim 1, 2, 3 or 4, which additionally contains beta carotene.

6. A mixture according to claim 1, 2, 3, 4 or 5, which additionally contains selenium.

7. A mixture according to claim 1, 2, 3, 4, 5 or 6, which additionally contains L-cysteine.

8. A mixture according to one of claims 1 to 7 as radical catcher (non-enzymatic scavenger) and radical chain terminator for the treatment of illnesses which develop with hyperradicalism, with the phenomenon of "oxidative stresses" (peroxidation) and/or with enzymopathy and enzymopenia of enzymes having an antioxidant effect.

**Revendications**

1. Mélange de substances, contenant
du gluthation réduit
et au moins une combinaison d'anthocyane du groupe
pélargonidine
péonidine
cyanidine
malvinidine
pétunidine
delphinidine
pour le traitement thérapeutique du corps humain ou animal.

2. Mélange de substances, contenant
au moins un dérivé thiol du glutathion du groupe
(thio)-éther-méthyl-glutathionyl
(thio)-éther-éthyl-glutathionyl
(thio)-ester-mono-acétyl-glutathionyl
(thio)-ester-mono-acide phosphorique-glutathionyl
et au moins une combinaison d'anthocyane du groupe
pélargonidine
péonidine
cyanidine
malvinidine
pétunidine
delphinidine

pour le traitement thérapeutique du corps humain ou animal.

3. Mélange de substances, contenant
du guthation réduit
et au moins un dérivé thiol du glutathion du groupe
(thio)-éther-méthyl-glutathionyl
(thio)-éther-éthyl-glutathionyl
(thio)-ester-mono-acétyl-glutathionyl
(thio)-ester-mono-acide phosphorique-glutathionyl
et au moins une combinaison d'anthocyane du groupe
pélargonidine
péonidine
cyanidine
malvinidine
pétunidine
delphinidine
pour le traitement thérapeutique du corps humain ou animal.

4. Mélange de substances selon la revendication 1, 2 ou 3, qui contient en plus de la vitamine E et/ou de la vitamine A.

5. Mélange de substances selon la revendication 1, 2, 3 ou 4, qui contient en plus de la $\beta$ -carotène.

6. Mélange de substances selon la revendication 1, 2, 3, 4 ou 5 qui contient en plus du sélénium.

7. Mélange de substances selon la revendication 1, 2, 3, 4, 5 ou 6 qui contient en plus de la L-cystéine.

8. Mélange de substances selon l'une des revendications 1 à 7 en tant que capteur de radicaux (Scavenger non-enzymatique) et terminateur de chaînes radicalaires pour le traitement d'affections qui s'accompagnent d'une hyperradicalie, du phénomène du "stress oxydant" (suroxydation) et/ou avec des enzymes agissant de manière anti-oxydante en cas d'enzymopénies et enzymopathies